## Europäisches Patentamt

## European Patent Office

## Office européen des brevets

(11) Veröffentlichungsnummer: **0 124 750**
**B1**

(12) **EUROPÄISCHE PATENTSCHRIFT**

(45) Veröffentlichungstag der Patentschrift:
03.09.86

(51) Int. Cl.⁴: **A 61 K 6/04, C 22 C 5/04**

(21) Anmeldenummer: **84103475.4**

(22) Anmeldetag: **29.03.84**

(54) **Dental-Legierung auf der Basis von Palladium zur Herstellung von festsitzendem und herausnehmbaren Zahnersatz.**

(30) Priorität: **06.05.83 DE 3316595**

(43) Veröffentlichungstag der Anmeldung:
**14.11.84 Patentblatt 84/46**

(45) Bekanntmachung des Hinweises auf die Patenterteilung:
**03.09.86 Patentblatt 86/36**

(84) Benannte Vertragsstaaten:
**AT CH DE FR LI NL**

(56) Entgegenhaltungen:
**DE-A-2 523 971**
**US-A-4 261 744**
**US-A-4 336 290**
**US-A-4 400 350**

(73) Patentinhaber: **Degussa Aktiengesellschaft, Weissfrauenstrasse 9, D-6000 Frankfurt am Main 1 (DE)**

(72) Erfinder: **Wagner, Ruldof, Dr. Dipl.- Ing., Breslauer Strasse 10, D-7537 Remchingen 3 (DE)**
Erfinder: **Schiwiora, Harry, Theodor- Heuss- Strasse 37, D-7530 Pforzheim (DE)**

EP 0 124 750 B1

**01 245 750**

**Beschreibung**

Die Erfindung betrifft eine Dentallegierung auf der Basis von Palladium zur Herstellung von festsitzendem und herausnehmbaren Zahnersatz. Dadurch kann sie gleichermaßen zum Aufbrennen von Dentalporzellan als auch als Modellgußlegierung sowie für kombinierten Zahnersatz verwendet werden.

Es ist bekannt, Prothesenelemente aus unedlen Co-Cr-Legierungen einerseits und festsitzenden Zahnersatz aus hochgoldhaltigen Legierungen mit Keramikverblendung andererseits gemeinsam im Mund einzusetzen. Bei dauerndem Kontakt von Werkstoffen mit unterschiedlichen Korrosionspotentialen können sich aber im Mund recht stabile Korrosionselemente bilden.

Es besteht deshalb der Wunsch, nur einen einzigen Werkstoff in der Mundhöhle einzusetzen. Versuche zum keramischen Verkleiden von Co-Cr-Legierungen sind bisher daran gescheitert, daß deren Aufbrenneigenschaften für Dentalporzellane nicht befriedigend waren; bei hochgoldhaltigen Legierungen lassen das hohe spezifische Gewicht, der geringere Elastizitätsmodul und auch der hohe Goldpreis eine Verwendung als Modellgußlegierung nicht für geeignet erscheinen.

Von der Korrosionsbeständigkeit, vom spezifischen Gewicht, von Materialkosten und von den mechanischen Eigenschaften wurden sich palladiumlegierungen als Werkstoff anbieten, mit dem sowohl Prothesenteile als auch Aufbrennarbeiten ausgeführt werden könnten.

Um die mechanische Stabilität im Munde und auch eine Deformationsresistenz beim Herausnehmen einer Prothese zu gewährleisten, sollten die dazu verwendeten Legierungen Dehngrenzen von mindestens 550 MPa und Bruchdehnungen von mindestens 4 % aufweisen (nach DIN 13 912).

Bisher bekanntgewordene Aufbrennlegierungen auf Palladiumbasis (z. B. DE-AS 24 40 425, US-PS 4336 290,US-PS 4 319 877 oder US-PS 4 179 288) erfüllen zwar die Anforderung bezüglich der Dehngrenze ($R_p$ 0,2), die Bruch-Dehnungswerte liegen jedoch bei maximal 2 - 3 %. Dies ist hauptsächlich auf das dendritische Gefüge zurückzuführen, das nur minimale Verformungen zuläßt.

Für die Brauchbarkeit einer Legierung zum Verkleiden mit dentalkeramischen Massen ist vor allem entscheidend, daß der Wärmeausdehnungskoeffizient (WAK) der Legierung im Bereich von Raumtemperatur bis 600° C an den der keramischen Massen angepaßt ist, und zwar so, daß er in diesem Temperaturbereich immer etwas größer ist.

Es war daher Aufgabe der Erfindung, Dentallegierungen auf der Basis Palladium zur Herstellung von festsitzendem und herausnehmbaren Zahnersatz zu finden, die sowohl eine genügend hohe Streckgrenze als auch eine ausreichend hohe Dehnung aufweisen und sich mit Dentalkeramik verblenden lassen.

Diese Aufgabe wurde erfindungsgemäß durch Dentallegierungen gelöst, die aus 65 - 85 % Palladium, 0 - 10 % Gold und/oder 0 - 5 % Platin, 0,1 - 10 % Zinn, 1 - 10 % Gallium, 1 - 12 % Kupfer sowie 0,05 - 1,5 % Ruthenium und/oder 0,05 - 0,7 % Rhenium besteht. Die Prozentangaben beziehen sich dabei auf Gewichtsprozente.

Vorteilhafterweise verwendet man Legierungen, die 65 bis 80 %, insbesondere 78 - 80 % Palladium, 0,5 bis 5 %, insbesondere 0,8 bis 2 % Gold, 0,5 bis 5 %, insbesondere 0,8 bis 2 % Platin, 3 bis 8 %, insbesondere 3 bis 5 % Kupfer, 4 bis 8 %, insbesondere 5 bis 6 % Gallium, 4 bis 9 %, insbesondere 6 bis 9 % Zinn und 0,2 bis 0,9 %, insbesondere 0,4 bis 0,6 % Rheniumoder 0,7 bis 0,9 % Ruthenium enthalten.

Die Festigkeit dieser Legierungen wird im wesentlichen durch die Gallium-Zugabe gesteuert und die für gute Dehnungseigenschaften notwendige Feinkörnigkeit durch die Zugabe von Platin und Ruthenium oder Rhenium erreicht. Der notwendige Wärmeausdehnungskoeffizient wird durch eine Kombination der Zusatzelemente Gold, Kupfer, Gallium und Zinn eingestellt.

Besonders bewährt haben sich Legierungen aus (in Gew.%)
1) 80% Pd, 1% Au, 1% Pt, 5% Cu, 6% Ga, 6,5% Sn, 0,5% Re
2) 80% Pd, 1,5% Au, 1,5% Pt, 3% Cu, 5% Ga, 8,5% Sn, 0,5% Re
3) 80% Pd, 1% Au, 1% Pt, 5% Cu, 6% Ga, 6,5% Sn, 0,5% Ru
4) 79,7% Pd, 1% Au, 1% Pt, 5% Cu, 6% Ga, 6,5% Sn, 0,8% Ru
5) 81,2 % Pd, 5 %, Cu, 6 % Ga, 7% Sn, 0,8% Ru
6) 70% Pd, 5% Au, 5% Pt, 4% Sn, 9% Cn, 6,3% Ga, 0,5% Ru, 0,2% Re.

Schmelzintervall, Härte, Streckgrenze, Bruchdehnung und Kornzahl dieser sechs Legierungen sind in der folgenden Tabelle angegeben.

Die erfindungsgemäßen Legierungen besitzen sehr gute Fließeigenschaften und weisen im Temperaturbereich 20 - 600°C einen Ausdehnungskoeffizienten zwischen 13,5 und 14,5 x $10^{-6/°}$C auf. Ihre mechanischen Eigenschaften sind durch Wärmebehandlung nur geringfügig veränderbar. Die Legierungen lassen sich sehr gut kaltverformen.

Wichtig ist die Anwesenheit von Zinn in den erfindungsgemäßen Legierungen, da dieses wie Gallium den Schmelzpunkt herabsetzt, im Gegensatz zu Gallium aber keine wesentliche Härtesteigerung bewirkt.

## Tabelle

| Le-gierung Nr. | Schmelzintervall (°C) | Vickershärte* (HV5/30) | 0,2 %-Dehngrenze* (MPa) | Bruchdehnung* (%) | Kornzahl (Körner/mm²) |
|---|---|---|---|---|---|
| 1 | 1290—1160 | 271 | 585 | 36,6 | ca. 6000 |
| 2 | 1320—1160 | 249 | 559 | 36,5 | ca. 6000 |
| 3 | 1280—1175 | 257 | 597 | 13,8 | ca. 2300 |
| 4 | 1290—1155 | 260 | 638 | 15,8 | ca. 3000 |
| 5 | 1290—1155 | 270 | 599 | 6,0 | ca. 4000 |
| 6 | 1255—1080 | 290 | 737 | 17,3 | ca. 3500 |

* gemessen an Prüfkörpern nach DIN 13 912

## Patentansprüche

1. Dentallegierung auf der Basis von Palladium zur Herstellung von festsitzendem und herausnehmbaren Zahnersatz, dadurch gekennzeichnet, daß sie aus 65 - 85 % Palladium, 0 - 10 % Gold und/oder 0 - 5 % Platin, 0,1 - 10 % Zinn, 1 - 10 % Gallium, 1 - 12 % Kupfer sowie 0,05 - 1,5 % Ruthenium und/oder 0,05 - 0,7 % Rhenium besteht.

2. Dentallegierung nach Anspruch 1, dadurch gekennzeichnet, daß sie aus 65 - 80 % Palladium, 0,5 - 5 % Gold, 0,5 - 5 % Platin, 3 - 8 % Kupfer, 4 - 8 % Gallium, 4 - 9 % Zinn und 0,2 - 0,7 % Rhenium oder 0,7 - 0,9 % Ruthenium besteht.

3. Dentallegierung nach Anspruch 1 und 2, dadurch gekennzeichnet, daß sie aus 78- 80 % Palladium, 0,8 - 2 % Gold, 0,8 - 2 % Platin, 3 - 5 % Kupfer, 5 - 6 % Gallium, 6 - 9 % Zinn und 0,4 - 0,6 Rhenium oder 0,7 bis 0,9 % Ruthenium besteht.

4. Dentallegierung nach Anspruch 1 bis 3, dadurch gekennzeichnet, daß sie aus 80 % Palladium, 1 % Gold, 1 % Platin, 5 % Kupfer, 6 % Gallium, 6,5 % Zinn und 0,5 % Rhenium besteht.

## Revendications

1°) Alliage dentaire à base de palladium pour la fabrication de dents artificielles fixes et de prothèses dentaires amovibles, caractérisé par le fait qu'il est constitué de 65-85 % de palladium, 0-10 % d'or et/ou 0-5 % de platine, 0,1-10 X d'étein, 1-10 % de gallium, 1-12 % de cuivre ainsi que de 0,05-3,5 % de rhuténium et/ou 0,05-0,7 % de rhénium.

2°) Alliage dentaire selon la revendication 1, caractérisé par le fait qu'il est constitué de 65-80 % de palladium, 0,5-5 % d'or, 0,5-5 % de platine, 3-8 % de cuivre, 4-8 % de gallium, 4-9 % d'étain et 0,2-0,7 % de rhénium ou 0,7-0,9 % de rhuténium.

3°) Alliage dentaire selon les revendications et 2, caractérisé par le fait qu'il est constitué de 78-80 % de palladium, 0,8-2 % d'or, 0,8-2 % de platine, 3-5 % de cuivre, 5-6 % de gallium, 6-9 % d'étain et 0,4-0,6 % de rhénium ou de 0,7 à 0,9 % de rhuténium.

4°) Alliage dentaire selon les revendications 1 à 3, caractérisé par le fait qu'il est constitué de 80 % de palladium, 1 % d'or, 1 % de platine, 5 % de cuivre, 6 % de palladium, 6,5 % d'étain et 0,5 % de rhénium.

## Claims

1. A dental alloy based on palladium for the production of fixed and removable dental restorations characterised in that it is composed of from 65 to 85% of palladium, from 0 to 10% of gold and/or from 0 to 5% of platinum, from 0.1 to 10% of tin, from 1 to 10% of gallium, from 1 to 12% of copper and from 0.05 to 1.5% of ruthenium and/or from 0.05 to 0.7% of rhenium.

2. A dental alloy according to claim 1, characterised in that it is composed of from 65 to 80% of palladium, from 0.5 to 5% of gold, from 0.5 to 5% of platinum, from 3 to 8% of copper, from 4 to 8% of gallium, from 4 to 9% of tin and from 0.2 to 0.7% of rhenium or from 0.7 to 0.9% of ruthenium.

3. A dental alloy according to claims 1 and 2, characterised in that it is composed of from 78 to 80% of palladium, from 0.8 to 2% of gold, from 0.8 to 2% of platinum, from 3 to 5% of copper, from 5 to 6% of gallium from 6 to 9% of tin and from 0.4 to 0.6% of rhenium or from 0.7 to 0.9% of ruthenium.

4. A dental alloy according to claims 1 to 3, characterised in that it is composed of 80% of palladium, 1% of gold, 1% of platinum, 5% of copper, 6% of gallium, 6.5% of tin and 0.5% of rhenium.